# EUROPÄISCHE PATENTSCHRIFT

(11) **EP 1 197 202 B1**
(45) Veröffentlichungstag und Bekanntmachung des Hinweises auf die Patenterteilung: **09.07.2003**
(21) Anmeldenummer: 01123314.5
(22) Anmeldetag: 08.10.2001
(51) Int. Cl.: A61K 7/13

(54) **Haarfärbemittel enthaltend 2,6-Diaminopurin**
Hair dye composition comprising 2,6-diaminopurine
Composition de coloration capillaire comprenant 2,6-diaminopurine

(30) Priorität: 14.10.2000 DE 10051041
(43) Veröffentlichungstag der Anmeldung: 17.04.2002
(73) Patentinhaber: KPSS-Kao Professional Salon Services GmbH, 64297 Darmstadt (DE)
(72) Erfinder: Lorenz, Heribert, 64401 Gross Bieberau (DE); Kaffenberger, Klaus, 64665 Alsbach-Hähnlein (DE)

(56) Entgegenhaltungen:
- DE-C- 19 724 337
- DE-C- 19 821 535
- DE-C- 19 834 651

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können und das Haar selbst bei kurzfristiger wiederholter Anwendung nicht schädigt.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht werden können.

Es wurde auch bereits vorgeschlagen, diese Lücke durch Verwendung alternativer Entwicklersubstanzen zu schließen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann Abstriche in der Farbintensität anderer Nuancen hingenommen werden.

Eine weitgehend optimale Lösung dieses Problems wird durch den in der EP-A 615 743 beschriebenen Einsatz von 2-(2'-Hydroxyethylamino)-5-aminotoluol bzw. dessen wasserlöslichen Salzen und die aus der EP-B 467 026 bekannten Triaminohydroxypyrimidine, insbesondere 2,5,6-Triamino-4-hydroxypyrimidin, 2,4,5-Triamino-6-hydroxypyrimidin, 4,5,6-Triamino-2-hydroxypyrimidin bzw. deren Salze, insbesondere die Sulfate, als Entwicklersubstanzen in Haarfärbemitteln erreicht. Selbst dadurch bleiben jedoch noch farbtechnische Wünsche offen.

Die Erfindung geht daher von der Aufgabenstellung aus, ein Haarfärbemittel zu schaffen, das zur Herstellung einer großen Anzahl von Farbtönen geeignet.ist und das Haar selbst bei kurzzeitiger wiederholter Anwendung nicht schädigt.

Diese Aufgabe wird dadurch gelöst, daß ein solches Haarfärbemittel ein mit Peroxid reagierendes Oxidationsfarbstoff-System enthält, das aus einer Kombination aus 2,6-Diaminopurin bzw. dessen wasserlöslichen Salzen und mindestens einer weiteren Kuppler- und/oder Entwicklersubstanz besteht.

Diese ist vorzugsweise ausgewählt aus der Gruppe 1-Methoxy-2-amino-4-(β-hydroxyethylamino)benzol, 2-Amino-3-hydroxypyridin, 2,5-Diaminopyridin, 2,6-Diaminopyridin, 3-Amino-2-methylamino-6-methoxypyridin, 2-(Dimethylamino)-5-aminopyridin, 1-(β-Hydroxyethyl)-2,5-diaminobenzol, 2-(2'-Hydroxyethylamino)-5-aminotoluol, 1-Amino-4-bis-(2'-hydroxyethyl)aminobenzol, 2,4,5,6-Tetraaminopyrimidin, 2,5,6-Triamino-4-hydroxypyrimidin, 2-Amino-N,N-diethylaminotoluol, 1,4-Diaminobenzol, 1,3-Diaminobenzol, 2,5-Diaminotoluol, Resorcin, 2-Methylresorcin, 4-Chlorresorcin, 2-Amino-4-chlorphenol, 1,6-Dihydroxynaphthalin, 1,7-Dihydroxynaphthalin, 2-Aminophenol, 3-Aminophenol, 1-Methyl-2-hydroxy-4-aminobenzol, 5-Amino-2-methoxyphenol, 2-Methyl-5-hydroxyethylaminophenol,4-Amino-3-methylphenol, 5-Amino-2-methylphenol, 2-Amino-4-ß-hydroxyethylaminoanisol bzw. deren wasserlösliche Salze und/oder 1-Naphthol.

Damit soll jedoch der Zusatz weiterer Entwickler- und Kupplersubstanzen keineswegs ausgeschlossen sein.

Bei Anwendung dieser Zusammensetzungen auf Basis einer üblichen Grundlage werden nach der Oxidation mit Peroxid sehr ausdrucksvolle, intensive, dauerhafte Haarfärbungen vor allem im Gelb-, Orange-, Gold- und Braunbereich erhalten, die durch Zusatz entsprechender weiterer Entwickler- und Kupplersubstanzen noch zu anderen Farbnuancen variiert werden können.

Auch die zusätzliche Mitverwendung weiterer, an sich bekannter Entwicklersubstanzen ist möglich. Neben den bereits oben genannten sind hierbei insbesondere noch 5-Aminosalicylsäure und/oder 1,2,4-Triaminobenzol zu erwähnen.

Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Das bevorzugte Gewichtsverhältnis von 2,6-Diaminopurin zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1.

Die Kupplersubstanz(en) als Reaktionspartner der Entwicklersubstanz(en) liegen in den erfindüngsgemäßen Haarfärbemitteln etwa im gleichen molaren Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 10 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Wasser | @ 100,00 |

Die erfindungsgemäßen Oxidationsfarbstoff-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

### Beispiel 1:

| | |
|---|---|
| 0,68 (Gew.-%) | 2,6-Diaminopurin (PC 92) |
| 0,55 | 1-Methyl-2-hydroxy-4-amino-benzol |

### Färbung:

Intensives Silbergelb.

### Beispiel 2:

| | |
|---|---|
| 0,68 (Gew.-%) | PC 92 |
| 0,91 | p-Toluylendiaminsulfat |

### Färbung:

Glänzendes Hellbraunorange.

### Beispiel 3:

| | |
|---|---|
| 0,68 (Gew.-%) | PC 92 |
| 0,50 | Resorcin |

### Färbung:

Intensives Gelbweiß.

### Beispiel 4:

| | |
|---|---|
| 0,68 (Gew.-%) | PC 92 |
| 1,07 | 2,4,5,6-Tetraaminopyrimidinsulfat |

### Färbung:

Intensives Graubraun.

### Beispiel 5:

| | |
|---|---|
| 0,68 (Gew.-%) | PC 92 |
| 0,49 | p-Phenylendiamin |

### Färbung:

Glänzendes Graubraunviolett.

### Beispiel 6:

| | |
|---|---|
| 0,68 (Gew.-%) | PC 92 |
| 0,49 | 3-Aminophenol |

### Färbung:

Intensives Weißgelb.

### Beispiel 7:

| | |
|---|---|
| 0,68 (Gew.-%) | PC 92 |
| 1,08 | 4-Hydroxy-2,5,6-triaminopyrimidinsulfat |

### Färbung:

Glänzendes Gelbweiß.

### Beispiel 8:

| | |
|---|---|
| 0,68 (Gew.-%) | PC 92 |
| 1,42 | 2-(2'-Hydroxyethylamino)-5-toluolosulfat |

### Färbung:

Intensives Hellgrauorange.

### Beispiel 9:

| | |
|---|---|
| 0,68 (Gew.-%) | PC 92 |
| 0,55 | 4-Amino-3-methylphenol |

### Färbung:

Glänzendes Graugelb.

### Beispiel 10:

| | |
|---|---|
| 0,68 (Gew.-%) | PC 92 |
| 0,25 | 2-Amino-4-β-hydroxyethylamino-anisolsulfat |

### Färbung:

Glänzendes Gelbgrau.

### Beispiel 11:

| | |
|---|---|
| 0,68 (Gew.-%) | PC 92 |
| 0,50 | 2-Amino-3-hydroxypyridin |

### Färbung:

Glänzendes Hellbraunorange.

### Beispiel 12:

| | |
|---|---|
| 0,68 (Gew.-%) | PC 92 |
| 1,98 | p-Toluylendiaminsulfat |
| 0,55 | 1-Methyl-2-hydroxy-4-aminobenzol |

### Färbung:

Glänzendes Purpurviolett.

## Patentansprüche

1. Haarfärbemittel auf Basis eines mit Peroxid reagierenden Oxidationsfarbstoff-Vorprodukts, enthaltend 2,6-Diaminopurin und/oder dessen wasserlösliche Salze und mindestens eine weitere Entwickler- und/oder Kupplersubstanz.

2. Haarfärbemittel nach Anspruch 1, enthaltend als weitere Entwickler- und Kupplersubstanz(en) mindestens eine Komponente, ausgewählt aus der Gruppe 1-Methoxy-2-amino-4-(β-hydroxyethylamino)benzol, 2,5-Diaminopyridin, 2,6-Diaminopyridin, 2-Amino-3-hydroxypyridin, 3-Amino-2-methylamino-6-methoxypyridin, 2-(Dimethylamino)-5-aminopyridin, 1-(β-Hydroxyethyl)-2,5-diaminobenzol, 2-(2'-Hydroxyethylamino)-5-aminotoluol, 1-Amino-4-bis-(2'-hydroxyethyl)-aminobenzol, 2,4,5,6-Tetraaminopyrimidin 2,5,6-Triamino-4-hydroxypyrimidin, 5-Amino-2-methylphenol, 2-Methyl-5-hydroxyethylaminophenol, 4-Amino-3-methylphenol, 2-Amino-5-N,N-diethylaminotoluol, 1,4- Diaminobenzol, 2,5-Diaminotoluol, 2-Amino-4-chlorphenol, 1,4-Diaminobenzol, 1,3-Diaminobenzol, 1-Methyl-2-hydroxy-4-aminobenzol, 1,6-Dihydroxynaphthalin, 1,7 Dihydroxynaphthalin, Resorcin, 2-Methylresorcin, 4-Chlorresorcin, 1-Naphthol, 2-Aminophenol, 5-Amino-2-methoxyphenol, 2-Amlno-4-β-hydroxyethylaminoanisol und/oder 3-Aminophenol bzw. deren wasserlöslichen Salzen.

## Claims

1. Hair dyeing composition on the basis of an oxidation dyestuff precursor reacting with peroxide, comprising 2.6-diaminopurine and/or the water-soluble salts thereof and at least one additional developing and/or coupling substance.

2. Hair dyeing composition according to claim 1, comprising as additional developing and coupling substance (s) at least one component selected from the group 1-methoxy-2-amino-4-(β-hydroxyethyl amino)benzene, 2.5-diaminopyridine, 2.6-di-amino-pyridine, 2-amino-3-hydroxypyridine, 3-amino-2-methyl amino-6-methoxy-pyridine, 2-(dimethylamino)-5-amino-pyridine, 1-(β-hydroxyethyl)-2.5-diamino-benzene, 2-(2'-hydroxyethyl amino)-5-aminotoluene, 1-amino-4-bis-(2'-hydroxy-ethyl)aminobenzene, 2.4.5.6-tetraminopyrimidine, 2.5.6-triamino-4-hydroxy-pyrimidine, 5-amino-2-methyl phenol, 2-methyl-5-hydroxyethyl aminophenol, 4-amino-3-methyl phenol, 2-amino-5-N,N-diethyl amino-toluene, 1.4-diamino-benzene, 2.5-diaminotoluene, 2-amino-4-chlorophenol, 1.4-diamino-benzene, 1.3-diaminobenzene, 1-methyl-2-hydroxy-4-aminobenzene, 1.6-dihydroxy-naphthaline, 1.7-dihydroxynaphthaline, resorcinol, 2-methyl resorcinol, 4-chlororesorcinol, 1-naphthol, 2-aminophenol, 5-amino-2-methoxyphenol, 2-amino-4-β-hydroxyethyl aminoanisole, and/or 3-amino-phenol or the water-soluble salts thereof.

## Revendications

1. Colorant capillaire à base d'un précurseur de colorant par oxydation, qui réagit avec un peroxyde, contenant de la 2,6-diaminopurine et/ou ses sels hydrosolubles et au moins une substance supplémentaire révélatrice et/ou de couplage.

2. Colorant capillaire selon la revendication 1, contenant à titre de substance(s) supplémentaire(s) révelatrice(s) et de couplage, au moins un composant choisi dans le groupe formé par le 1-méthoxy-2-amino-4-(β-hydroxyéthylamino)benzène, la 2,5-diaminopyridine, la 2,6-diaminopyridine, la 2-amino-3-hydroxypyridine, la 3-amino-2-méthylamino-6-méthoxypyridine, la 2-(diméthylamino)-5-aminopyridine, le 1-(β-hydroxyéthyl)-2,5-diaminobenzène, le 2-(2'-hydroxyéthylamino)-5-aminotoluène, le 1-amino-4-bis(2'-hydroxyéthyl)-aminobenzène, la 2,4,5,6-tétraaminopyrimidine, la 2,5,6-triamino-4-hydroxypyrimidine, le 5-amino-2-méthylphonol, le 2-méthyl-5-hydroxyéthylaminophénol, le 4-amino-3-méthylphénol, le 4-amino-3-méthylphénol, le 2-amino-5-N,N-diéthylaminotoluène, le 1,4-diaminobenzène, le 2,5-diaminotoluène, le 2-amino-4-chlorophénol, le 1,4-diaminobenzène, le 1,3-diaminobenzène, le 1-méthyl-2-hydroxy-4-aminobenzène, le 1,6-dihydroxynaphtalène, le 1,7-dihydroxynaphtalène, la résorcine, la 2-méthylrésorcine, la 4-chlororésorcine, le 1-naphtol, le 2-aminophénol, le 5-amino-2-méthoxyphénol, le 2-amino-4-β-hydroxyéthylaminoanisol et/ou le 3-aminophénol ou leurs sels hydrosolubles.
